# EUROPÄISCHE PATENTSCHRIFT

(11) **EP 2 930 525 B1**
(45) Veröffentlichungstag und Bekanntmachung des Hinweises auf die Patenterteilung: **04.12.2019**
(21) Anmeldenummer: 15160572.2
(22) Anmeldetag: 24.03.2015
(51) Int. Cl.: G01R 33/50, G01R 33/563, G01R 33/565

(54) **DYNAMISCHE MR-BILDGEBUNG MIT VARIABLEM KONTRAST**
DYNAMIC MR IMAGING WITH VARIABLE CONTRAST
IMAGERIE DYNAMIQUE RM À CONTRASTE VARIABLE

(30) Priorität: 08.04.2014 DE 102014206724
(43) Veröffentlichungstag der Anmeldung: 14.10.2015
(73) Patentinhaber: Siemens Healthcare GmbH, 91052 Erlangen (DE)
(72) Erfinder: Stalder, Aurélien, 91052 Erlangen (DE); Greiser, Andreas, 91054 Erlangen (DE); Schmidt, Michaela, 91080 Uttenreuth (DE); Speier, Peter, 91056 Erlangen (DE); Zenge, Michael, 90419 Nürnberg (DE)

(56) Entgegenhaltungen:
- DE-A1-102012 206 585
- US-A1- 2012 189 183
- US-A1- 2013 272 591
- PETER KELLMAN ET AL: "Extracellular volume fraction mapping in the myocardium, part 1: evaluation of an automated method", JOURNAL OF CARDIOVASCULAR MAGNETIC RESONANCE, BIOMED CENTRAL LTD, LONDON UK, Bd. 14, Nr. 1, 10. September 2012 (2012-09-10), Seite 63, XP021116179, ISSN: 1532-429X, DOI: 10.1186/1532-429X-14-63
- TSAI JYUN-MING ET AL: "Free-breathing MOLLI: Application to myocardial Tmapping", MEDICAL PHYSICS, AIP, MELVILLE, NY, US, Bd. 39, Nr. 12, 1. Dezember 2012 (2012-12-01), Seiten 7291-7302, XP012160761, ISSN: 0094-2405, DOI: 10.1118/1.4764915 [gefunden am 2012-11-26]
- SANTINI F ET AL: "Fast simultaneous T1 and T2 mapping of the heart", PROCEEDINGS OF THE INTERNATIONAL SOCIETY FOR MAGNETIC RESONANCE IN MEDICINE, ISMRM, 21ST ANNUAL MEETING AND EXHIBITION, SALT LAKE CITY, UTAH, USA, 20-26 APRIL 2013, 7. April 2013 (2013-04-07), Seite 4561, XP040632157,

## Beschreibung

Die vorliegende Erfindung betrifft ein Verfahren zur Berechnung von MR-Bildern eines Untersuchungsobjekts das eine zyklische Bewegung ausführt und eine MR-Anlage hierfür.

Bei der Aufnahme von MR-Bilder von sich bewegenden Organen muss die Eigenbewegung des Organs, beispielsweise des Herzens, berücksichtigt werden und evtl. die Bewegung des gesamten Organs aufgrund der Bewegung der Umgebung. Dies ist bei der Bewegung des Herzens oder der Leber bei der Atmung der Fall, wobei diese zweite Bewegung als repetitive und annähernd periodische Bewegung vorausgesetzt wird. Eine erste Möglichkeit für die Bildgebung von sich bewegenden Objekten ist die sogenannte Single-Shot-Technik, bei der der Rohdatenraum vollständig nach Einstrahlen eines HF-Pulses ausgelesen wird und bei der die Aufnahme der Daten schnell genug ist, um die Bewegung einzufrieren. Die weitere Aufnahmemöglichkeit ist die segmentierte Aufnahmetechnik, bei der die Datenaufnahme für ein Bild auf mehrere Bewegungszyklen aufgeteilt wird und MR-Daten nur in vergleichbaren Bewegungsphasen aufgenommen werden. Bei der Herzbildgebung muss die Atmung und die Herzbewegung berücksichtigt werden. Die Bewegung durch Atmung kann durch Atemanhaltetechniken minimiert werden oder durch Navigator-Gating eingefroren werden. Die erste Möglichkeit begrenzt die Messdauer, und die zweite Möglichkeit begrenzt die Effizienz und erhöht die Komplexität.

Eine Möglichkeit der Datenaufnahme ist die sogenannte CINE-Datenaufnahme für die Messung der Herzmuskelbewegung, bei der viele MR-Bilder pro Herzzyklus aufgenommen werden, wobei ein guter konstanter Kontrast zwischen Herzmuskel und Blut benötigt wird. Dies bedeutet die Verwendung einer Sequenz mit einem guten T2/T1-Kontrast. Eine andere Möglichkeit der Bildaufnahme ist eine statische Gewebecharakterisierung, bei der Gewebeeigenschaften bestimmt werden durch Messung eines Bildes pro Herzschlag. Hierbei erfolgt üblicherweise ein Präparationsblock wie ein Sättigungspuls oder Inversionspuls oder eine T2-Präparierung gefolgt von einer optionalen Wartezeit und der nachfolgenden Bildaufnahme. Durch den Präparationsblock wird der zur Charakterisierung benötigte Kontrast erzeugt.

Weiterhin ist die Verwendung von Kontrastmitteln bekannt, wobei die Gewebecharakterisierung mit oder ohne Kontrastmittelverwendung durchgeführt werden kann. Klinisch von Bedeutung sind insbesondere T1-Kontraste vor und nach Kontrastmittelgabe. Eine wichtige Gewebecharakterisierung am Herzen ist die Narbendarstellung durch ein sogenanntes Delayed-Enhancement, bei dem fünf bis zehn Minuten nach Kontrastmittelgabe ein T1-gewichtetes Bild derart aufgenommen wird, dass das gesunde Myocard kein Signal mehr abgibt, die Narbe jedoch ein helles Signal liefert.

Aus der DE 10 2012 206 585 A1 ist ein Verfahren zur schnellen ortsaufgelösten Bestimmung eines Magnetresonanz-Relaxationsparameters eines Herzens eines Untersuchungsobjekts bekannt, wobei eine Bewegungskorrektur mit Hilfe von synthetischen Schätzbildern auf eine vorgegebene Herzphase als Referenz durchgeführt wird.

Wünschenswert wäre es nun, eine Aufnahmetechnik zu erhalten, bei der eine Bewegungsdarstellung eines sich bewegenden Organs und eine Gewebecharakterisierung mit dem benötigten Kontrast kombiniert werden kann.

Diese Aufgabe wird durch die Merkmale der unabhängigen Ansprüche gelöst. Weitere Ausführungsformen sind in den abhängigen Ansprüchen beschrieben.

Gemäß einem ersten Aspekt der Erfindung wird ein Verfahren zur Berechnung von MR-Bildern eines Untersuchungsobjekts bereitgestellt, welches eine zyklische Bewegung ausführt, wobei MR-Signale zur Aufnahme von MR-Bildern des Untersuchungsobjekts über zumindest zwei Zyklen der zyklischen Bewegung detektiert werden und wobei in jedem der zumindest zwei Zyklen eine Vielzahl von MR-Bildern aufgenommen wird. Hierbei nähert sich die die MR-Bilder beeinflussende Magnetisierung des Untersuchungsobjekts über die zumindest zwei Zyklen einem Gleichgewichtszustand, wobei in einem zweiten Zyklus der zumindest zwei Zyklen die Magnetisierung näher am Gleichgewichtszustand ist als in einem ersten Zyklus der zumindest zwei Zyklen. Weiterhin wird eine Bewegungsinformation für verschiedene Bewegungsphasen der zyklischen Bewegung des Untersuchungsobjekts unter Verwendung der Vielzahl von MR-Bildern aus dem zweiten Zyklus derart bestimmt, dass eine Bewegungsinformation des Untersuchungsobjekts für jede der verschiedenen Bewegungsphasen bestimmt wird. Anschließend wird eine Bewegungskorrektur des Untersuchungsobjekts in den MR-Bildern des ersten Zyklus für die verschiedenen Bewegungsphasen der zyklischen Bewegung unter Verwendung der im zweiten Zyklus bestimmten Bewegungsinformation durchgeführt und zwar derart, dass für die verschiedenen MR-Bilder des ersten Zyklus jeweils ein bewegungskorrigiertes MR-Bild für die verschiedenen Bewegungsphasen der zyklischen Bewegung berechnet wird.

Durch die Verwendung der MR-Bilder im zweiten Zyklus, in der die Magnetisierung nahe am Gleichgewichtszustand ist, kann die Bewegungsinformation zuverlässig bestimmt werden, da die Kontraständerung in dieser Reihe von MR-Bildern des zweiten Zyklus nunmehr gering ist. Somit ist es möglich, die Bewegungsinformation, die beispielsweise eine Deformationsinformation sein kann, zuverlässig mit den MR-Bildern im zweiten Zyklus zu berechnen. Diese Bewegungsinformation wird dann auf die MR-Bilder im ersten Zyklus übertragen, bei dem die Magnetisierung weiter vom Gleichgewichtszustand weg ist. Dies bedeutet, dass sich die einzelnen MR-Bilder im ersten Zyklus stärker im Kontrast unterscheiden. Da nun mit Hilfe der Bewegungsinformation bewegungskorrigierte MR-Bilder für die verschiedenen Bewegungsphasen der zyklischen Bewegung berechnet werden, erhält man MR-Bilder für die verschiedenen Bewegungsphasen der zyklischen Bewegung und für verschiedene Kontraste. Weiterhin ist es nicht mehr notwendig vor der Bildaufnahme zu definieren, welchen Kontrast oder welche Phase der zyklischen Bewegung von Interesse ist, da diese Auswahl retrospektiv nach der Aufnahme der MR-Bilder bestimmt werden kann, da MR-Bildserien für verschiedene Kontraste berechnet wurden.

Die Anwendung der Bewegungsinformation aus dem zweiten Zyklus ist hierbei nicht auf einen einzigen ersten früheren Zyklus beschränkt. Die Bewegungsinformation aus dem zweiten Zyklus kann auch auf MR-Bilder aus mehreren vor diesem zweiten Zyklus liegenden Zyklen angewandt werden. Anders ausgedrückt kann die Bewegungsinformation des zweiten Zyklus auf die MR-Bilder von zumindest einem zeitlich davor liegenden Zyklus angewandt werden. Weiterhin können unvollständige Zyklen herangezogen werden, das heißt es müssen nicht alle MR-Bilder eines Zyklus verwendet werden. Weiterhin kann der Startpunkt der Zyklen bzw. der Startpunkt innerhalb der Zyklen für die Bewegungsbestimmung frei wählbar sein.

Für die Berechnung ist es möglich, dass für jede der verschiedenen Bewegungsphasen des Untersuchungsobjekts in dem zweiten Zyklus eine Bewegungsänderung relativ zu jeder der anderen Bewegungsphasen in dem zweiten Zyklus bestimmt wird. Eine Bewegungskorrektur kann dann in den MR-Bildern des ersten Zyklus derart durchgeführt werden, dass für jede Bewegungsphase der zyklischen Bewegung ein bewegungskorrigiertes MR-Bild im ersten Zyklus bestimmt wird und zwar für jedes MR-Bild des ersten Zyklus. Durch diese Bewegungsinformation von jeder Bewegungsphase zu allen anderen Bewegungsphasen können dann aus der Bewegungsinformation und den im ersten Zyklus aufgenommenen MR-Bildern MR-Bilder für die verschiedenen Bewegungsphasen im ersten Zyklus berechnet werden, die dann auch unterschiedliche Kontraste haben.

Hierbei ist vorzugsweise jedem MR-Bild ein Kontrastwert zugeordnet, mit dem das Untersuchungsobjekt in dem zugehörigen MR-Bild dargestellt ist. Hierbei ist eine Kontraständerung zwischen zeitlich benachbarten MR-Bildern des ersten Zyklus größer als bei zeitlich benachbarten MR-Bildern des zweiten Zyklus. Da sich die Magnetisierung bei der Aufnahme der MR-Bilder im zweiten Zyklus näher am Gleichgewichtszustand befindet als im ersten Zyklus, sind die Kontrastunterschiede zwischen den einzelnen MR-Bildern im ersten Zyklus größer als im zweiten Zyklus. Wird nun angenommen, dass jedes im ersten Zyklus aufgenommene MR-Bild einen unterschiedlichen Kontrastwert hat und jedes aufgenommene MR-Bild des ersten Zyklus einer Bewegungsphase zugeordnet werden kann, so ergibt sich für die verschiedenen Kontrastwerte des ersten Zyklus jeweils zumindest ein MR-Ausgangsbild, das bei dem zugehörigen Kontrastwert als MR-Bild aufgenommen wurde. Für die verschiedenen Kontrastwerte des ersten Zyklus wird nun unter Verwendung des bei dem zugehörigen Kontrastwert aufgenommenen MR-Bildes, das bei einer der Bewegungsphasen aufgenommen wurde, und unter Verwendung der Bewegungsinformation für die verschiedenen Bewegungsphasen bewegungskorrigierte MR-Bilder für die anderen fehlenden Bewegungsphasen berechnet. Diese haben dann den gleichen Kontrastwert wie das zugehörige Ausgangsbild.

Die im ersten Zyklus aufgenommenen MR-Bilder haben jeweils einen unterschiedlichen Kontrastwert. Für einen bestehenden Kontrastwert können dann mit Hilfe der im zweiten Zyklus gewonnenen Bewegungsinformation und dem aufgenommenen MR-Bild im ersten Zyklus, dem Ausgangsbild, die bewegungskorrigierten MR-Bilder für die jeweiligen anderen Bewegungsphasen berechnet werden. Somit liegt für diesen einen Kontrastwert eine Reihe von MR-Bildern vor, die die Bewegung des Untersuchungsobjekts bei gleichem Kontrast zeigen. Wenn dies für die anderen Kontrastwerte der Bilder des ersten Zyklus wiederholt wird, so erhält man eine Bildfolge für die verschiedenen Kontrastwerte, sodass beliebig für verschiedene Kontrastwerte aus der Bildfolge sogenannte bewegte oder CINE-Aufnahmen erstellt werden können.

Nach der Berechnung der bewegungskorrigierten MR-Bilder liegen MR-Bilder für alle Kontrastwerte des ersten Zyklus und für alle Bewegungsphasen der zyklischen Bewegung vor, die entweder aufgenommen oder berechnet wurden. Damit können nach der Aufnahme für beliebige Kontrastwerte MR-Bilder der zyklischen Bewegung betrachtet werden.

In einer Ausführungsform wird die Magnetisierung des Untersuchungsobjekts vor der Detektion der MR-Signale durch Einstrahlen eines Präparationspulses präpariert, wobei sich die Magnetisierung dann über die zumindest zwei Zyklen dem Gleichgewichtszustand nähert. Vor dem Start der eigentlichen Bildgebung mit dem Schalten der Gradienten- und HF-Pulse kann eine Präparierung beispielsweise durch einen Inversionspuls erfolgen, indem die Magnetisierung um 180° invertiert wird. Erfolgt dann die Bildaufnahme mit schnellen Gradientenechosequenzen, beispielsweise einer bSSFP-Sequenz (balanced steadystate free precession), so unterscheidet sich der Kontrast in den einzelnen MR-Bildern am Anfang, dem ersten Zyklus direkt nach Einstrahlung stark, während er sich im zweiten Zyklus nur noch geringfügig von MR-Bild zu MR-Bild ändert.

Anstelle der Präparierung mit einem Inversionspuls kann auch ein Sättigungspuls eingestrahlt werden, indem die Magnetisierung vor der Aufnahme der MR-Signale gesättigt wird und abschließend wieder in den Gleichgewichtszustand läuft. Es ist jedoch nicht die Einstrahlung eines Inversions- oder Sättigungspulses notwendig. In einer anderen Ausführungsform ist ein derartiger Verlauf der Magnetisierung erreichbar, wenn beispielsweise eine Gradientenechosequenz immer wieder mit einer Repetitionszeit TR verwendet wird, die geringer als die T1-Zeit ist. Auch hierbei läuft die Magnetisierung nach einer Weile in einen Gleichgewichtzustand.

Vorzugsweise ist der erste Zyklus der zeitlich erste Zyklus in der zyklischen Bewegung, in dem die MR-Signale des Untersuchungsobjekts aufgenommen werden, wobei der zweite Zyklus vorzugsweise der letzte Zyklus ist, in dem die MR-Signale des Untersuchungsobjekts aufgenommen werden. Im letzten Zyklus ist die Magnetisierung sehr nahe am Gleichgewichtszustand, während sich die Magnetisierung im ersten zeitlichen Zyklus noch am stärksten ändert.

Wenn das Untersuchungsobjekt beispielsweise das Herz ist, können die MR-Signale über mehrere Herzzyklen aufgenommen werden, beispielsweise eine Zahl zwischen 3 und 6, vorzugsweise 4 oder 5. Da ein Herzzyklus ungefähr eine Sekunde dauert, ist die Aufnahme der MR-Bilder innerhalb von drei bis ungefähr sechs Sekunden möglich. Während dieser Zeitspanne können auch nicht gesunde Untersuchungspersonen die Luft anhalten. Je nach Anwendungsgebiet können auch mehr als 6 Zyklen verwendet werden. Notwendig ist nur eine Zykluszahl ≥ 2. Für eine Aufnahme von vielen MR-Bildern innerhalb eines Herzzyklus ist es möglich die MR-Bilder mit Hilfe von Beschleunigungsverfahren, beispielsweise Compressed-Sensing-Technologie aufzunehmen. Diese Technik verkürzt, wie bekannt, weiterhin die Echozeit, indem gewisse Bedingungen der aufgenommenen MR-Daten ausgenutzt werden, um die Anzahl der wirklich aufgenommenen MR-Rohdatenpunkte zu reduzieren.

Weiterhin ist es möglich, aus den zu den verschiedenen Kontrastwerten berechneten und aufgenommenen MR-Bildern sogenannte ortsaufgelöste T1- und T2-Relaxationskarten des Untersuchungsobjekts zu erstellen. Wurde beispielsweise ein Inversionspuls verwendet, so kann mit dem Intensitätsverlauf in den einzelnen MR-Bildern auf die T1- bzw T2-Zeit geschlossen werden. Diese kann dann pixelweise für die verschiedenen Bewegungsphasen berechnet und dargestellt werden.

Die Erfindung betrifft ebenso eine MR-Anlage zur Berechnung der MR-Bilder mit einer Aufnahmeeinheit zur Aufnahme der MR-Signale und Erstellung der MR-Bilder über die zumindest zwei Zyklen der zyklischen Bewegung, wie oben erläutert, und mit einer Recheneinheit, die, wie oben ausführlich erklärt, die Bewegungsinformation für die verschiedenen Phasen der zyklischen Bewegung mit Hilfe der MR-Bilder aus dem zweiten Zyklus berechnet und auf die MR-Bilder im ersten Zyklus anwendet, um für die MR-Bilder im ersten Zyklus, die verschiedene Kontrastwerte haben, MR-Bilder für die verschiedenen Bewegungsphasen der Bewegung zu berechnen.

Weitere Vorteile und Ausgestaltungen der Erfindung ergeben sich aus der nachfolgend angeführten Beschreibung von Ausführungsbeispielen. Hierbei zeigen:
Fig. 1 eine MR-Anlage mit der die Aufnahme und Berechnung von MR-Bildern mit variablem Kontrast in kurzen Zeitspannen möglich ist,
Fig. 2 schematisch ein Schema, wie die Berechnung der bewegungskorrigierten MR-Bilder erfolgt,
Fig. 3 ein Schema zur Erläuterung der Berechnung der Bewegungsinformation für die verschiedenen Bewegungsphasen,
Fig. 4 eine Matrix mit der Darstellung der berechneten und aufgenommenen MR-Bilder in Abhängigkeit von der Bewegungsphase und dem Kontrast,
Fig. 5 ein Flussdiagramm mit Schritten, die bei der Berechnung der MR-Bilder mit variablem Kontrast durchgeführt werden.

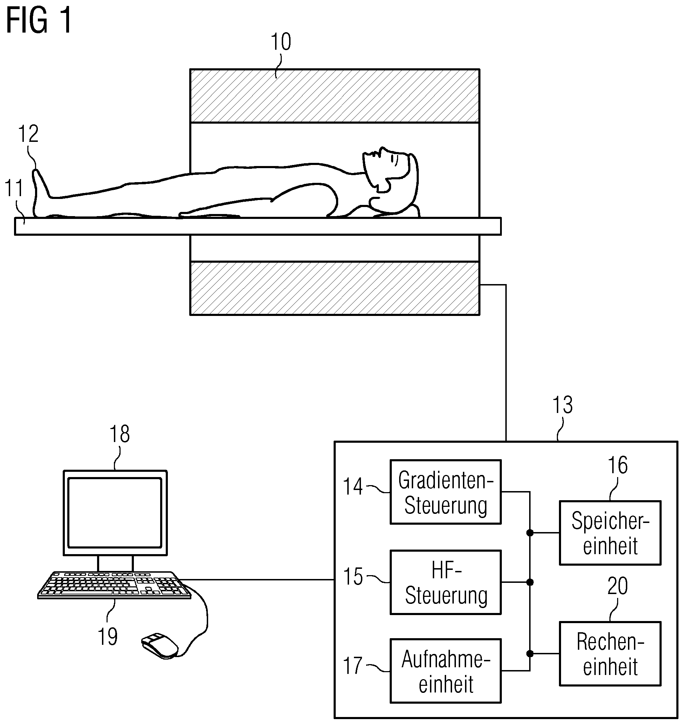

Fig. 1 zeigt schematisch eine Magnetresonanzanlage, mit der erfindungsgemäß MR-Bilder eines Untersuchungsobjekts, wie eines Organs, dass eine zyklische Bewegung ausführt, bei unterschiedlichen Kontrasten erstellt werden können. Die Magnetresonanzanlage weist einen Magnet 10 zur Erzeugung eines Polarisationsfeldes Bₒ auf, wobei eine auf einer Liege 11 angeordnete Untersuchungsperson 12 in das Zentrum des Magneten gefahren wird, um dort ortskodierte Magnetresonanzsignale aus einem Untersuchungsobjekt aufzunehmen. Durch Einstrahlen von Hochfrequenzpulsfolgen und Schalten von Magnetfeldgradienten kann die durch das Polarisationsfeld B₀ erzeugte Magnetisierung aus der Gleichgewichtslage ausgelenkt werden und die sich ergebende Magnetisierung kann mit nicht gezeigten Empfangsspulen in Magnetresonanzsignalen detektiert werden. Die allgemeine Funktionsweise zur Erstellung von Magnetresonanzsignalen mit verschiedenen Bildgebungssequenzen ist dem Fachmann bekannt, sodass auf eine detaillierte Erläuterung verzichtet wird.

Das Magnetresonanzgerät weist weiterhin eine zentrale Steuereinheit 13 auf, die zur Steuerung des MR-Geräts verwendet wird. Die zentrale Steuereinheit 13 weist eine Gradientensteuerung 14 zur Steuerung und Schaltung der Magnetfeldgradienten auf. Eine HF-Steuerung 15 ist vorgesehen zur Steuerung und Einstrahlung der HF-Pulse zur Auslenkung der Magnetisierung. In einer Speichereinheit 16 können beispielsweise die für die Aufnahme der MR-Bilder notwendigen Bildgebungssequenzen abgespeichert werden sowie andere Programme, die zum Betrieb der MR-Anlage notwendig sind. Eine Aufnahmeeinheit 17 steuert die Bildaufnahme und steuert damit in Abhängigkeit von der gewählten Bildgebungssequenz, in welcher Abfolge Magnetfeldgradienten und HF-Pulse eingestrahlt werden. Damit steuert die Aufnahmeeinheit 17 auch die Gradientensteuerung 14 und die HF-Steuerung 15. Die in einer Recheneinheit 20 berechneten MR-Bilder können auf einer Anzeige 18 angezeigt werden, und eine Bedienperson kann über eine Eingabeeinheit 19 die MR-Anlage bedienen.

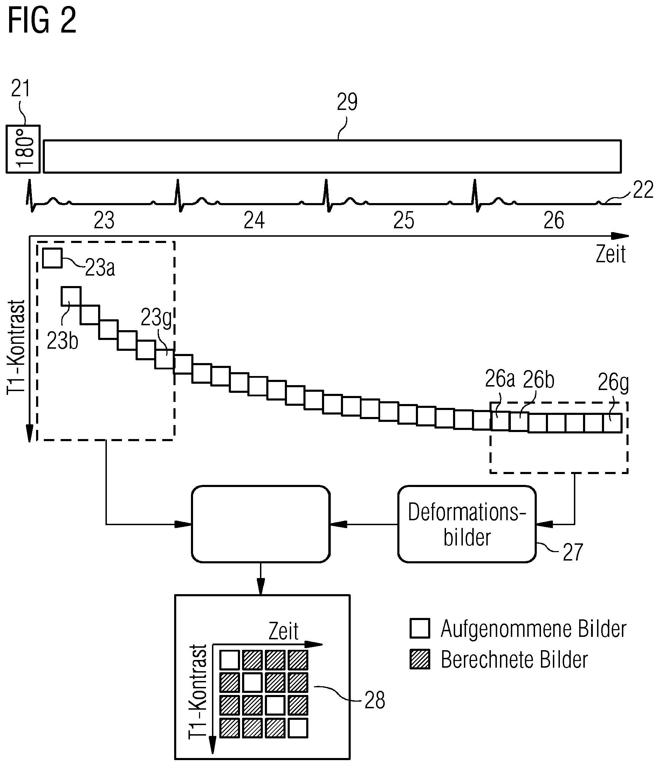

In Fig. 2 werden schematisch ein Teil der Bildgebungssequenz und die Nachbearbeitungsschritte dargestellt, mit denen Aufnahmen des Herzens, beispielsweise sogenannte Delayed-Enhancement-Untersuchungen, nach Kontrastmittelgabe durchgeführt werden können. Nach Einstrahlen eines HF-Pulses 21, hier eines Inversionspulses, der EKG-getriggert durch die EKG-Triggerung 22 erfolgt, erfolgt die Bildaufnahme während einer Zeitspanne, die schematisch mit dem Balken 29 dargestellt ist. Bei der Bildaufnahme kann es sich beispielsweise um eine bSSFP-Sequenz handeln, die eine Compressed-Sensing-Technologie mit einer k-t-Regularisierung verwendet. Beispielsweise werden 2D-MR-Daten aufgenommen nach dem Inversionspuls 21, der direkt nach der R-Zacke des EKGs eingestrahlt wird. Im dargestellten Fall erfolgt die Bildaufnahme über vier Herzzyklen: einem Herzzyklus 23, einem Herzzyklus 24, einem Herzzyklus 25 und einem Herzzyklus 26. Die zeitliche Auflösung der während der verschiedenen Herzzyklen aufgenommenen MR-Bilder kann zwischen 30 und 40 ms liegen, sodass pro Herzzyklus mehrere MR-Bilder aufgenommen werden.

In Fig. 2 ist weiterhin der Kontrast schematisch dargestellt, den die einzelnen MR-Bilder in den einzelnen Herzzyklen haben. Wie zu erkennen ist, ändert sich der Kontrast zwischen den einzelnen MR-Bildern 23a-23g des ersten Herzzyklus 23 sehr stark, aufgrund des gerade eingestrahlten Inversionspulses. Die Magnetisierung nähert sich über die Aufnahmezeit ihrem Gleichgewichtszustand, sodass im Zyklus 26 der Unterschied in der Magnetisierung zwischen den einzelnen MR-Bildern nur noch gering ist.

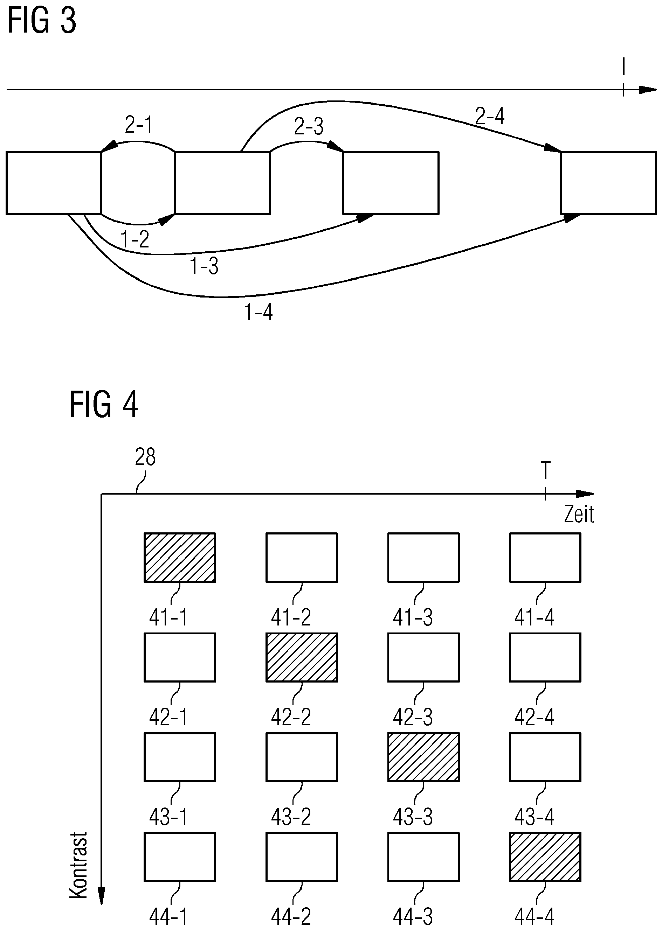

Die Aufnahme erfolgt über zumindest zwei Zyklen, wobei in einem ersten Zyklus, dem Zyklus 23, die Magnetisierungsänderung von MR-Bild zu MR-Bild größer ist als in einem zweiten Zyklus, im dargestellten Fall dem Zyklus 26. Die MR-Bilder 26a-26g des Zyklus 26 werden nun verwendet, um eine Bewegungsinformation des sich bewegenden Herzens zu berechnen, beispielsweise eine Deformationsinformation. Da die einzelnen MR-Bilder 26a-26g einen geringen Kontrastunterschied aufweisen, kann die Herzbewegung mit diesen Bildern gut bestimmt werden, da keine gewebebedingten Kontrastunterschiede zwischen den einzelnen Bildern auftreten. Wie eine Registrierung der einzelnen MR-Bilder bei verschiedenen Herzphasen zueinander möglich ist und wie daraus einzelne Deformationsbilder berechnet werden können, die die Deformation des Herzens in den einzelnen Herzphasen zeigen, ist dem Fachmann bekannt und wird hier nicht näher erläutert. Eine mögliche Berechnung der Bewegungsinformation ist beschrieben in "EFFICIENT SYMMETRIC AND INVERSE-CONSISTENT DEFORMABLE REGISTRATION THROUGH INTERLEAVED OPTIMIZATION", Christoph Guetter, Hui Xue, Christophe Chefd'hotel, Jens Guehring, Biomedical Imaging: From Nano to Macro, 2011 IEEE International Symposium, Seiten: 590 - 593, ISSN: 1945-7928. Diese Deformationsbilder, die aus den MR-Bildern des zweiten Zyklus, hier des letzten Zyklus 26, gewonnen wurden, sind in Fig. 2 schematisch in dem Feld 27 dargestellt. Damit ist die Herzbewegung identifiziert und kann auf die MR-Bilder 23a-23g, die im ersten Herzzyklus aufgenommen wurden, angewandt werden. Wie später noch weiter im Detail im Zusammenhang mit Figs. 3 und 4 erläutert werden wird, ist es somit möglich aus den Deformationsinformationen für die einzelnen Kontraste des ersten Zyklus jeweils MR-Bilder für die unterschiedlichen Phasen der zyklischen Bewegung zu bestimmen, wie es schematisch durch die Matrix 28 dargestellt ist.

Die oben beschriebene Deformationsinformation kann auf der Eigenbewegung des Herzens beruhen. Falls noch eine Restbewegung aufgrund des nicht vollständig angehaltenen Atems verbleibt, d.h. eine Bewegung durch die Bewegung der Umgebung, kann diese ebenfalls noch korrigiert werden. Wenn zwischen den beiden Zyklen 23 und 26 noch eine leichte Bewegung vorliegt, beispielsweise durch geringe Atemtätigkeit, kann diese kompensiert werden vor der Bestimmung der Bewegungsinformation durch Registrieren der MR-Bilder der verschiedenen Zyklen miteinander. Hierbei kann beispielsweise das letzte Bild aus dem ersten Zyklus, das Bild 23g, auf das letzte Bild des zweiten Zyklus, Bild 26g, registriert werden. Die sich hier ergebende zweite Bewegungsinformation kann dann auf die gesamten MR-Bilder des zweiten Zyklus angewandt werden. Allgemein können MR-Bilder der gleichen Bewegungsphase aus den beiden Zyklen miteinander verglichen werden, um daraus eine zweite Bewegungsinformation zu berechnen.

Anhand von Fig. 3 wird zunächst erläutert, welche Bewegungs- bzw. Deformationsinformation bestimmt wird. In Fig. 3 sind hierfür schematisch die verschiedenen Bewegungsphasen eines Herzzyklus dargestellt, wobei im dargestellten Fall vier Bewegungsphasen dargestellt sind. Selbstverständlich ist es möglich, die zyklische Bewegung in mehr oder weniger unterschiedliche Bewegungsphasen einzuteilen. Die einzelnen MR-Bilder 26a-26g werden den einzelnen Bewegungsphasen zugeordnet oder jedes Bild repräsentiert eine Bewegungsphase, wobei für jede Bewegungsphase zumindest ein MR-Bild vorliegt. Bei dem in Fig. 2 dargestellten Beispiel wurden pro Zyklus acht Bilder aufgenommen. Diese Zahl kann jedoch variieren und wurde nur zu Zeichnungszwecken verwendet. Wenn das oder die MR-Bilder der ersten Bewegungsphase mit den MR-Bildern der zweiten Bewegungsphase verglichen werden, so lässt sich die Bewegungsänderung bestimmen, die sich von der ersten relativ zur zweiten Bewegungsphase ergeben hat. In Fig. 3 ist dies schematisch mit dem Pfeil 1-2 dargestellt. Ebenso lässt sich die Bewegungs- oder Deformationsänderung von der ersten relativ zur dritten Phase bestimmen, im Bild dargestellt mit 1-3, und die Bewegungsänderung von der ersten zur vierten Phase ist mit 1-4 dargestellt. Weiterhin wird die Bewegungsänderung von der zweiten Phase relativ zur ersten Phase bzw. von der zweiten relativ zur dritten oder vierten Phase berechnet, sodass die Bewegungsänderung von jeder der Bewegungsphasen zu jeder der anderen Bewegungsphasen berechnet wird. Wird die Bewegung in n unterschiedliche Bewegungsphasen innerhalb des Zyklus eingeteilt, so ergeben sich n(n-1) Bewegungs- oder Deformationsinformationen. Diese Bewegungs- oder Deformationsinformation kann Translations- und/oder Rotationskomponenten beinhalten. Mit der derart bestimmten Bewegungsinformation ist es nun möglich für die MR-Bilder des ersten Zyklus MR-Bilder für die verschiedenen Bewegungsphasen zu bestimmen.

Dies ist näher in Fig. 4 erläutert, wo die Matrix 28 von Fig. 2 noch einmal im Detail dargestellt ist. Die in Fig. 4 schraffiert dargestellten MR-Bilder sind MR-Bilder, die in dem jeweiligen Zyklus von der MR-Anlage aufgenommen wurden, im dargestellten Fall 41-1, 42-2, 43-3 und 44-4. Diese vier MR-Bilder können beispielsweise irgendwelche der MR-Bilder 23a-23g von Fig. 2 sein. Das aufgenommene MR-Bild 41-1 hat einen ersten Kontrast, beispielsweise da es direkt nach Einstrahlen des Inversionspulses aufgenommen wurde. Mit Hilfe der Deformationsbilder, die, wie in Fig. 3 erläutert, berechnet wurden, können nun die MR-Bilder 41-2, 41-3 und 41-4 berechnet werden. Bezug nehmend auf das Beispiel von Fig. 3 würde hierbei die Deformationsinformation 1-2, 1-3 und 1-4 verwendet werden, um ausgehend von dem aufgenommenen MR-Bild 41-1 die Bilder 41-2 bis 41-4 zu berechnen. Somit steht für einen ersten Kontrast eine Abfolge von MR-Bildern bereit, die beispielsweise für eine CINE-Darstellung des sich bewegenden Herzens bei dem ersten Kontrast verwendet werden können. Eine ähnliche Berechnung ist für die MR-Bilder 42 möglich. Ausgehend von dem aufgenommenen MR-Bild und der Bewegungsinformation, hier der Bewegungsinformation 2-1, 2-3 und 2-4, werden die MR-Bilder 42-1, 42-3 und 42-4 berechnet, sodass für einen weiteren anderen Kontrast eine Abfolge von MR-Bildern berechnet wurde. Auf gleiche Weise können die MR-Bilder 43-1 bis 43-4 und 44-1 bis 44-3 berechnet werden. Wie in Fig. 4 schematisch zu erkennen ist, erhält man dadurch für unterschiedliche Kontrastwerte jeweils eine Abfolge von MR-Bildern zu unterschiedlichen Bewegungsphasen. Diese Abfolgen von MR-Bildern können von einem Arzt begutachtet werden, um bei den verschiedenen Kontrastwerten Informationen für die Beweglichkeit des Myokards zu gewinnen.

Weiterhin ist es möglich, aus der Magnetisierungsentwicklung, wie sie in Fig. 2 gezeigt ist, T1- und T2-Werte zu berechnen, beispielsweise durch einen 3-Parameter-Fit, der voxelbasiert berechnet wird auf Grundlage der erzeugten Bilder.

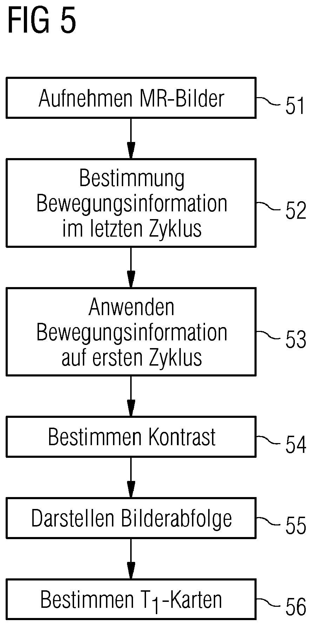

In Fig. 5 sind die einzelnen Schritte schematisch zusammengefasst. Es folgt die Aufnahme der MR-Bilder in Schritt 51, wobei, wie in Fig. 2 gezeigt wurde, vor der Aufnahme der MR-Bilder ein Inversionspuls oder ein Präparationspuls geschaltet werden kann. Wie eingangs erwähnt ist jedoch ein Inversions- oder Präparationspuls nicht absolut notwendig zur Erzeugung eines Magnetisierungsverlaufs, der sich dem Gleichgewichtszustand annähert. In Schritt 52 kann anschließend eine Bewegungsinformation aus den MR-Bildern des zweiten der zumindest zwei Zyklen bestimmt werden, wobei dies im Beispiel von Fig. 4 der vierte Zyklus war bzw. der letzte während der Aufnahme der MR-Bilder. In Schritt 53 wird diese Bewegungsinformation auf die MR-Bilder des ersten Zyklus angewandt, um, wie in Fig. 4 gezeigt, die MR-Bilder bei den verschiedenen Kontrastwerten für die verschiedenen Bewegungsphasen der zyklischen Bewegung zu berechnen. Anschließend kann nach der Bildaufnahme durch einen Anwender in Schritt 54 ein Kontrast bestimmt werden, bei dem der Anwender die Darstellung wünscht. Dies bedeutet, dass erst im Nachhinein und nicht vor Aufnahme der MR-Bilder im Schritt 51 ein beliebiger Kontrast gewählt wurde. In Schritt 55 kann dann die mit dem gewählten Kontrast gewünschte Bildfolge dargestellt werden, beispielsweise für eine CINE-Darstellung. Optional ist es möglich, in Schritt 56 aus dem Magnetisierungsverlauf, wie in Fig. 2 gezeigt, für die einzelnen Pixel T1- und T2-Werte zu berechnen durch Anfitten einer Kurve.

Bei der oben beschriebenen Ausführungsform wurde die Bewegungsinformation des letzten Zyklus, des sogenannten zweiten Zyklus, nur auf die MR-Bilder des ersten Zyklus angewandt. Es ist jedoch auch möglich, dass Bezug nehmend auf Fig. 2 die Bewegungsinformation des letztes Zyklus auch auf die MR-Bilder des zweiten Zyklus, den Zyklus 24, anzuwenden, bei dem wiederum andere Kontrastwerte auftreten als im ersten Zyklus 23. Zwar sind die Kontrastunterschiede im Zyklus 24 nicht mehr so groß wie im Zyklus 23, jedoch ist die Verwendung der Bewegungsinformation nicht auf die MR-Bilder des ersten Zyklus 23 beschränkt; eine Anwendung auf die ersten Zyklen 23, 24 ist ebenso denkbar.

Zusammenfassend ermöglicht die oben beschriebene Erfindung die Erstellung von MR-Bildern mit einer ausreichenden räumlichen und zeitlichen Auflösung bei verschiedenen Kontrasten.

## Patentansprüche

1. Verfahren zur Berechnung von MR-Bildern eines Untersuchungsobjekts, das eine zyklische Bewegung ausführt, mit den folgenden Schritten:
- Detektieren von MR-Signalen zur Aufnahme von MR-Bildern des Untersuchungsobjekts über zumindest 2 Zyklen (23-26) der zyklischen Bewegung , wobei in jedem der zumindest 2 Zyklen eine Vielzahl von MR-Bildern aufgenommen wird, wobei eine die MR-Bilder beeinflussende Magnetisierung des Untersuchungsobjekts sich über die zumindest 2 Zyklen (23-26) einem Gleichgewichtszustand nähert und in einem zweiten Zyklus (26) der zumindest 2 Zyklen näher an dem Gleichgewichtszustand ist als in einem ersten Zyklus (23) der zumindest 2 Zyklen,
- Bestimmen einer Bewegungsinformation für verschiedene Bewegungsphasen der zyklischen Bewegung des Untersuchungsobjekts unter Verwendung der Vielzahl der MR-Bilder (26a-26g) aus dem zweiten Zyklus (26) derart, dass eine Bewegungsinformation des Untersuchungsobjekts für jede der verschiedenen Bewegungsphasen bestimmt wird,
- Durchführen einer Bewegungskorrektur des Untersuchungsobjekts in den MR-Bildern (23a-23g) des ersten Zyklus (23) für die verschiedenen Bewegungsphasen der zyklischen Bewegung unter Verwendung von der im zweiten Zyklus bestimmten Bewegungsinformation derart, dass für die verschiedenen MR-Bilder des ersten Zyklus jeweils ein bewegungskorrigiertes MR-Bild für die verschiedenen Bewegungsphasen der zyklischen Bewegung berechnet wird.

2. Verfahren nach Anspruch 1, **dadurch gekennzeichnet, dass** für jede der verschiedenen Bewegungsphasen des Untersuchungsobjekts in dem zweiten Zyklus eine Bewegungsänderung relativ zu jeder der anderen Bewegungsphasen in dem zweiten Zyklus bestimmt wird, und eine Bewegungskorrektur in den MR-Bildern (23a-23g) des ersten Zyklus derart durchgeführt wird, dass für jede Bewegungsphase der zyklischen Bewegung ein bewegungskorrigiertes MR-Bild im ersten Zyklus bestimmt wird.

3. Verfahren nach einem der vorstehenden Ansprüche, **dadurch gekennzeichnet, dass** jedem MR-Bild ein Kontrastwert zugeordnet ist, mit dem das Untersuchungsobjekt in dem zugehörigen MR-Bild dargestellt ist, wobei eine Kontraständerung zwischen zeitlich benachbarten MR-Bildern des ersten Zyklus größer ist als bei zeitlich benachbarten MR-Bildern des zweiten Zyklus.

4. Verfahren nach einem der vorstehenden Ansprüche, **dadurch gekennzeichnet, dass** jedes im ersten Zyklus aufgenommene MR-Bild (23a-23g) einen unterschiedlichen Kontrastwert hat, und jedes aufgenommene MR-Bild (23a-23g) des ersten Zyklus einer Bewegungsphase zugeordnet wird, so dass sich für die verschiedenen Kontrastwerte des ersten Zyklus jeweils zumindest ein MR-Ausgangsbild ergibt, das bei dem zugehörigen Kontrastwert als MR-Bild aufgenommen wurde, wobei für die verschiedenen Kontrastwerte des ersten Zyklus, unter Verwendung des bei dem zugehörigen Kontrastwert aufgenommenen MR-Ausgangsbildes, das bei einer der Bewegungsphasen aufgenommen wurde, und unter Verwendung der Bewegungsinformation für die verschiedenen Bewegungsphasen, bewegungskorrigierte MR-Bilder für die anderen fehlenden Bewegungsphasen berechnet werden, die den gleichen Kontrastwert haben wie das zugehörige Ausgangsbild.

5. Verfahren nach Anspruch 4, **dadurch gekennzeichnet, dass** nach der Berechnung der bewegungskorrigierten MR-Bilder MR-Bilder für alle Kontrastwerte des ersten Zyklus und für alle Bewegungsphasen der zyklischen Bewegung vorliegen, die entweder aufgenommen oder berechnet wurden.

6. Verfahren nach einem der vorstehenden Ansprüche, **dadurch gekennzeichnet, dass** die Magnetisierung des Untersuchungsobjekts vor dem Detektieren der MR-Signale durch Einstrahlen eines Präparationspulses (21) präpariert wird und sich während der Detektion der MR-Signale in den zumindest 2 Zyklen dem Gleichgewichtszustand nähert.

7. Verfahren nach Anspruch 6, **dadurch gekennzeichnet, dass** der Präparationspuls ein Inversionspuls ist, der die Magnetisierung invertiert.

8. Verfahren nach Anspruch 6 oder 7, **dadurch gekennzeichnet, dass** der erste Zyklus (23) der zeitlich erste Zyklus nach Einstrahlen des Präparationspulses (21) ist, in dem die MR-Signale des Untersuchungsobjekts aufgenommen werden, wobei der zweite Zyklus (26) zeitlich nach dem ersten Zyklus folgt.

9. Verfahren nach einem der Ansprüche 1 bis 5, **dadurch gekennzeichnet, dass** die Magnetisierung sich während der Detektion der MR-Signale dem Gleichgewichtszustand nähert, indem während der Aufnahme der MR-Bilder mehrere HF-Pulse in das Untersuchungsobjekt eingestrahlt werden in einem zeitlichen Abstand, der geringer ist als die T1 Zeit des Untersuchungsobjekts.

10. Verfahren nach einem der vorstehenden Ansprüche, **dadurch gekennzeichnet, dass** der erste Zyklus (23) der zeitlich erste Zyklus in der zyklischen Bewegung ist, in dem die MR-Signale des Untersuchungsobjekts aufgenommen werden, wobei der zweite Zyklus (26) der letzte Zyklus ist, in dem die MR-Signale des Untersuchungsobjekts aufgenommen werden.

11. Verfahren nach einem der vorstehenden Ansprüche, **dadurch gekennzeichnet, dass** das Untersuchungsobjekt das Herz ist, wobei die MR-Signale über eine Anzahl Herzzyklen aufgenommen werden, die zwischen 3 und 6 liegt.

12. Verfahren nach einem der vorstehenden Ansprüche, **dadurch gekennzeichnet, dass** die MR-Bilder mit Hilfe von Beschleunigungsverfahren aufgenommen werden.

13. Verfahren nach einem der Ansprüche 4 bis 12, **dadurch gekennzeichnet, dass** die aufgenommenen MR-Bilder und die berechneten bewegungskorrigierten MR-Bilder verwendet werden zur Berechnung von ortsaufgelösten T1- und T2-Relaxationszeiten des Untersuchungsobjekts.

14. Verfahren nach einem der vorstehenden Ansprüche, **dadurch gekennzeichnet, dass** die Bewegungsinformation eine erste Bewegungsinformation ist, die die Bewegung eines Organs als Untersuchungsobjekt berücksichtigt, wobei eine zweite Bewegungsinformation berechnet wird, die die Bewegung des Organs durch die Bewegung der Umgebung berücksichtigt, wobei die zweite Bewegungsinformation bestimmt wird durch Registrieren von einem MR-Bild des ersten Zyklus auf ein MR-Bild des zweiten Zyklus, wobei beide MR-Bilder der gleichen Bewegungsphase angehören.

15. MR-Anlage zur Berechnung von MR-Bildern eines Untersuchungsobjekts, das eine zyklische Bewegung ausführt, welche aufweist:
- eine Aufnahmeeinheit (17) die ausgebildet ist, MR-Signale zur Aufnahme von MR-Bildern des Untersuchungsobjekts über zumindest 2 Zyklen der zyklischen Bewegung zu detektieren und MR-Bilder zu rekonstruieren, wobei die Aufnahmeeinheit (17) ausgebildet ist, in jedem der zumindest 2 Zyklen eine Vielzahl von MR-Bildern aufzunehmen, wobei eine die MR-Bilder beeinflussende Magnetisierung des Untersuchungsobjekts sich über die zumindest 2 Zyklen einem Gleichgewichtszustand nähert und in einem zweiten Zyklus (26) der zumindest 2 Zyklen näher an dem Gleichgewichtszustand ist als in einem ersten Zyklus (23) der zumindest 2 Zyklen,
- eine Recheneinheit (20), die ausgebildet ist, eine Bewegungsinformation für verschiedene Bewegungsphasen der zyklischen Bewegung des Untersuchungsobjekts unter Verwendung der Vielzahl der MR-Bilder (26a-26g) aus dem zweiten Zyklus derart zu bestimmen, dass eine Bewegungsinformation des Untersuchungsobjekts für jede der verschiedenen Bewegungsphasen bestimmt wird, wobei die Recheneinheit (20) ausgebildet ist, eine Bewegungskorrektur des Untersuchungsobjekts in den MR-Bildern des ersten Zyklus für die verschiedenen Bewegungsphasen der zyklischen Bewegung unter Verwendung von der im zweiten Zyklus bestimmten Bewegungsinformation derart durchzuführen, dass für die verschiedenen MR-Bilder des ersten Zyklus jeweils ein bewegungskorrigiertes MR-Bild für die verschiedenen Bewegungsphasen der zyklischen Bewegung berechnet wird.

## Claims

1. Method for computing MR images of an examination object which performs a cyclic movement, with the following steps:
- Detecting MR signals for recording MR images of the examination object over at least 2 cycles (23-26) of the cyclic movement, wherein, in each of the at least 2 cycles, a plurality of MR images is recorded, wherein, over the at least 2 cycles (23-26), a magnetisation of the examination object influencing the MR images approaches a state of equilibrium and, in a second cycle (26) of the at least 2 cycles, is closer to the state of equilibrium than in a first cycle (23) of the at least 2 cycles,
- Determining movement information for various movement phases of the cyclic movement of the examination object using the plurality of MR images (26a-26g) from the second cycle (26) such that movement information of the examination object for each of the various movement phases is determined,
- Carrying out a movement correction of the examination object in the MR images (23a-23g) of the first cycle (23) for the various movement phases of the cyclic movement using the movement information determined in the second cycle such that, for the various MR images of the first cycle, a movement-corrected MR image is computed in each case for the various movement phases of the cyclic movement.

2. Method according to claim 1, **characterised in that**, for each of the various movement phases of the examination object in the second cycle a movement change relative to each of the other movement phases in the second cycle is determined, and a movement correction is carried out in the MR images (23a-23g) of the first cycle such that a movement-corrected MR image in the first cycle is determined for each movement phase of the cyclic movement.

3. Method according to one of the preceding claims, **characterised in that** each MR image is assigned a contrast value with which the examination object is displayed in the associated MR image, wherein a contrast change between temporally-adjacent MR images of the first cycle is greater than for temporally-adjacent MR images of the second cycle.

4. Method according to one of the preceding claims, **characterised in that** each MR image (23a-23g) recorded in the first cycle has a different contrast value, and each recorded MR image (23a-23g) of the first cycle is assigned a movement phase, so that for the various contrast values of the first cycle in each case, at least one initial MR image is produced, that was recorded at the associated contrast value as an MR image, wherein for the various contrast values of the first cycle, using the initial MR image recorded at the associated contrast value, which was recorded for one of the movement phases, and using the movement information for the various movement phases, movement-corrected MR images for the other missing movement phases are computed, which have the same contrast value as the associated initial image.

5. Method according to claim 4, **characterised in that**, after the computation of the movement-corrected MR images, MR images for all contrast values of the first cycle and for all movement phases of the cyclic movement are present, which have either been recorded or computed.

6. Method according to one of the preceding claims, **characterised in that** the magnetisation of the examination object is prepared before the detection of the MR signals by applying a preparation pulse (21) and during the detection of the MR signals in the at least 2 cycles approaches the state of equilibrium.

7. Method according to claim 6, **characterised in that** the preparation pulse is an inversion pulse which inverts the magnetisation.

8. Method according to claim 6 or 7, **characterised in that** the first cycle (23) is the first cycle in time after application of the preparation pulse (21), in which the MR signals of the examination object are recorded, wherein the second cycle (26) follows on from the first cycle in time.

9. Method according to one of claims 1 to 5, **characterised in that** the magnetisation approaches its state of equilibrium during the detection of the MR signals, **in that** during the recording of the MR images a number of RF pulses are applied to the examination object at an interval which is smaller than the T1 time of the examination object.

10. Method according to one of the preceding claims, **characterised in that** the first cycle (23) is the first cycle in time in the cyclic movement, in which the MR signals of the examination object are recorded, wherein the second cycle (26) is the last cycle in which the MR signals of the examination object are recorded.

11. Method according to one of the preceding claims, **characterised in that** the examination object is the heart, wherein the MR signals are recorded over a number of heart cycles which lie between 3 and 6.

12. Method according to one of the preceding claims, **characterised in that** the MR images are recorded with the aid of acceleration methods.

13. Method according to one of claims 4 to 12, **characterised in that** the recorded MR images and the computed movement-corrected MR images are used for computing of locally-resolved T1 and T2 relaxation times of the examination object.

14. Method according to one of the preceding claims, **characterised in that** the movement information is first movement information which takes account of the movement of an organ as examination object, wherein second movement information is computed, which takes account of the movement of the organ by the movement of its surroundings, wherein the second movement information is determined by registration of an MR image of the first cycle to an MR image of the second cycle, wherein both MR images belong to the same movement phase.

15. MR apparatus for computing MR images of an examination object which executes a cyclic movement, which has:
- A recording unit (17) which is embodied to detect MR signals for recording MR images of the examination object over at least 2 cycles of the cyclic movement and to reconstruct MR images, wherein the recording unit (17) is embodied, in each of the at least 2 cycles, to record a plurality of MR images, wherein a magnetisation of the examination object influencing the MR images approaches a state of equilibrium over the at least 2 cycles and in a second cycle (26) of the at least 2 cycles is closer to the state of equilibrium than in a first cycle (23) of the at least 2 cycles,
- A processing unit (20) which is embodied to determine movement information for various movement phases of the cyclic movement of the examination object using the plurality of MR images (26a-26g) from the second cycle such that movement information of the examination object is determined for each of the various movement phases, wherein the processing unit (20) is embodied to carry out a movement correction of the examination object in the MR images of the first cycle for the various movement phases of the cyclic movement using the movement determined in the second cycle such that a movement-corrected MR image is computed for the various MR images of the first cycle in each case for the various movement phases of the cyclic movement.

## Revendications

1. Procédé de calcul d'images RM d'un objet à examiner, qui effectue un déplacement cyclique, comprenant les stades suivants :
- on détecte des signaux RM pour l'enregistrement d'images RM d'un objet à examiner sur au moins deux cycles (23 à 26) du déplacement cyclique, dans lequel, dans chacun des au moins deux cycles, on enregistre une pluralité d'images RM, une aimantation, influençant les images RM de l'objet à examiner, se rapprochant, sur les au moins deux cycles (23 à 26), d'un état d'équilibre et, dans un deuxième cycle (26) des au moins deux cycles, étant plus proche de l'état d'équilibre que dans un premier cycle (23) des au moins deux cycles,
- on détermine une information de déplacement pour diverses phases du déplacement cyclique de l'objet à examiner, en utilisant la pluralité des images RM (26a-26g) du deuxième cycle (26), de manière à déterminer une information de déplacement de l'objet à examiner pour chacune des phases différentes du déplacement,
- on effectue une correction du déplacement de l'objet à examiner dans les images RM (23a-23g) du premier cycle (23) pour les diverses phases du déplacement cyclique, en utilisant l'information de déplacement déterminée dans le deuxième cycle, de manière à calculer, pour les diverses images RM du premier cycle, respectivement, une image RM corrigée en déplacement pour les diverses phases du déplacement cyclique.

2. Procédé suivant la revendication 1, **caractérisé en ce que**, pour chacune des diverses phases du déplacement de l'objet à examiner dans le deuxième cycle, on détermine une modification du déplacement par rapport à chacune des autres phases du déplacement dans le deuxième cycle et on effectue une correction du déplacement dans les images RM (23a-23g) du premier cycle, de manière à déterminer, pour chaque phase du déplacement cyclique, une image RM corrigée en déplacement dans le premier cycle.

3. Procédé suivant l'une des revendications précédentes, **caractérisé en ce que** l'on associe, à chaque image RM, une valeur de contraste, par laquelle l'objet à examiner est représenté dans l'image RM associée, une variation de contraste entre des images RM voisines dans le temps du premier cycle étant plus grande que pour des images RM voisines dans le temps du deuxième cycle.

4. Procédé suivant l'une des revendications précédentes, **caractérisé en ce que** chaque image RM (23a-23g) enregistrée dans le premier cycle a une valeur de contraste différente et on associe chaque image RM (23a-23g) enregistrée du premier cycle à une phase de déplacement, de manière à obtenir, pour les diverses valeurs de contraste du premier cycle, respectivement, au moins une image initiale RM, qui a été enregistrée comme image RM à la valeur de contraste associée, dans lequel on calcule, pour les diverses valeurs de contraste du premier cycle, en utilisant l'image initiale RM enregistrée à la valeur de contraste associée, qui a été enregistrée à l'une des phases de déplacement, et en utilisant l'information de déplacement pour les diverses phases de déplacement, des images RM corrigées en déplacement pour les autres phases manquantes du déplacement, qui ont la même valeur de contraste que l'image initiale associée.

5. Procédé suivant la revendication 4, **caractérisé en ce qu'**après le calcul des images RM corrigées en déplacement, il y a des images RM pour toutes les valeurs de contraste du premier cycle et pour toutes les phases du déplacement cyclique, qui ont été enregistrées ou calculées.

6. Procédé suivant l'une des revendications précédentes, **caractérisé en ce que** l'on prépare l'aimantation de l'objet à examiner avant la détection des signaux RM par incidence d'une impulsion (21) de préparation et l'aimantation se rapproche de l'état d'équilibre pendant la détection des signaux RM dans les au moins deux cycles.

7. Procédé suivant la revendication 6, **caractérisé en ce que** l'impulsion de préparation est une impulsion d'inversion, qui inverse l'aimantation.

8. Procédé suivant la revendication 6 ou 7, **caractérisé en ce que** le premier cycle (23) est le premier cycle dans le temps après l'incidence de l'impulsion (21) de préparation, dans lequel les signaux RM de l'objet à examiner sont enregistrés, le deuxième cycle (26) faisant suite dans le temps au premier cycle.

9. Procédé suivant l'une des revendications 1 à 5, **caractérisé en ce que** l'aimantation se rapproche de l'état d'équilibre pendant la détection des signaux RM par le fait que, pendant l'enregistrement des images RM, plusieurs impulsions HF sont incidentes sur l'objet à examiner dans une distance dans le temps, qui est plus petite que le temps T1 de l'objet à examiner.

10. Procédé suivant l'une des revendications précédentes, **caractérisé en ce que** le premier cycle (23) est le premier cycle dans le temps dans le déplacement cyclique, dans lequel les signaux RM de l'objet à examiner sont enregistrés, le deuxième cycle (26) étant le dernier cycle dans lequel les signaux RM de l'objet à examiner sont enregistrés.

11. Procédé suivant l'une des revendications précédentes, **caractérisé en ce que** l'objet à examiner est le cœur, les signaux RM étant enregistrés sur un nombre de cycles cardiaques compris entre 3 et 6.

12. Procédé suivant l'une des revendications précédentes, **caractérisé en ce que** l'on enregistre les images RM à l'aide de procédés d'accélération.

13. Procédé suivant l'une des revendications 4 à 12, **caractérisé en ce que** l'on utilise les images RM enregistrées et les images RM calculées corrigées en déplacement pour calculer des temps de relaxation T1 et T2 à résolution spatiale de l'objet à examiner.

14. Procédé suivant l'une des revendications précédentes, **caractérisé en ce que** l'information de déplacement est une première information de déplacement, qui prend en compte le déplacement d'un organe comme objet à examiner, dans lequel on calcule une deuxième information de déplacement, qui prend en compte des déplacements de l'organe par le déplacement de ce qui l'entoure, dans lequel on détermine la deuxième information de déplacement par enregistrement d'une image RM du premier cycle sur une image RM du deuxième cycle, les deux images RM appartenant à la même phase de déplacement.

15. Installation RM de calcul d'image RM d'un objet à examiner, qui effectue un déplacement cyclique, l'installation comprenant :
- une unité (17) d'enregistrement, constituée pour détecter des signaux RM pour l'enregistrement d'images RM de l'objet à examiner sur au moins deux cycles du déplacement cyclique et pour reconstruire des images RM, l'unité (17) d'enregistrement étant constituée pour enregistrer dans chacun des au moins deux cycles une pluralité d'images RM, une aimantation influençant les images RM de l'objet à examiner se rapprochant d'un état d'équilibre sur les au moins deux cycles et étant, dans un deuxième cycle (26) des au moins deux cycles, plus proche de l'état d'équilibre que dans un premier cycle (23) des au moins deux cycles,
- une unité (20) de calcul, constituée pour déterminer une information de déplacement pour diverses phases du déplacement cyclique de l'objet à examiner, en utilisant la pluralité des images RM (26a-26g) du deuxième cycle, de manière à déterminer une information de déplacement de l'objet à examiner pour chacune des diverses phases du déplacement, l'unité (20) de calcul étant constituée pour effectuer une correction du déplacement de l'objet à examiner dans les images RM du premier cycle pour les diverses phases du déplacement cyclique, en utilisant l'information de déplacement déterminée dans le deuxième cycle, de manière à calculer, pour les diverses images RM du premier cycle, respectivement, une image RM corrigée en déplacement pour les diverses phases du déplacement cyclique.
